# EUROPEAN PATENT SPECIFICATION

(11) **EP 2 219 632 B1**
(45) Date of publication and mention of the grant of the patent: **03.04.2019**
(21) Application number: 08850783.5
(22) Date of filing: 30.10.2008
(51) Int. Cl.: A61K 31/10, A61K 31/7004, A61K 31/7008, A61P 17/02

(54) **COMPOSITION FOR THE TREATMENT OF HYPERTROPHIC SCARS COMPRISING FUCOSE, DIMETHYLSULFONE AND ACETYL GLUCOSAMINE**
ZUSAMMENSETZUNG ZUR BEHANDLUNG HYPERTROPHER NARBEN MIT FUCOSE, DIMETHYLSULFON UND ACETYLGLUCOSAMIN
COMPOSITION RENFERMANT DU FUCOSE, DE LA DIMÉTHYLSULFONE ET DE L'ACÉTYL-GLUCOSAMINE, DESTINÉE AU TRAITEMENT DE CICATRICES HYPERTROPHIQUES

(30) Priority: 15.11.2007 IT BS20070178
(43) Date of publication of application: 25.08.2010
(73) Proprietor: Exalya S.r.l., 25010 San Felice del Benaco (Brescia) (IT)
(72) Inventor: de Paoli Ambrosi, Gianfranco, 25087 Salò (Brescia) (IT)
(74) Representative: Sangiacomo, Fulvia
(86) International application number: PCT/IT2008/000675
(87) International publication number: WO 2009/063522

(56) References cited:
- EP-A- 0 852 946
- EP-A- 1 749 532
- WO-A-02/28400
- WO-A-03/045366
- WO-A-2005/117913
- FR-A- 2 853 539
- PETERSZEGI G ET AL: "Protection by L-fucose and fucose-rich polysaccharides against ROS-produced cell death in presence of ascorbate." BIOMEDICINE AND PHARMACOTHERAPY, vol. 57, no. 3-4, May 2003 (2003-05), pages 130-133, XP002517047 ISSN: 0753-3322
- ISNARD N ET AL: "Studies on corneal wound healing: Effect of fucose on iodine vapor-burnt rabbit corneas" OPHTHALMOLOGICA 200511 CH, vol. 219, no. 6, November 2005 (2005-11), pages 324-333, XP002517048 ISSN: 0030-3755

## Description

### Field of the Invention

This invention concerns a new composition, both for pharmaceutical use and in a medical device form, as well as for cosmetics, able to obstruct the sclerotic fibre process, to prevent the formation of hypertrophic and scars and already formed keloids, as well as aiding the cicatrisation process, improving the biomechanical properties of the cutis (such as elasticity and stretchability), restraining cicatrisation retraction phenomena.

### State of the Art

The physiological response of the organism to a continual loss of cutaneous solution is characterized by a complex mechanism of articulated events between them, which only in recent years has been almost fully cleared up.

Independently from the type of wound, whether acute or chronic, and the entity of the loss of tissue, the healing of each wound proceeds in phases that overlap over time and which cannot be separated one from the other.

Repair of the tissue is a dynamic and interactive process that takes place normally in our organism and that involves soluble mediators, extracellular matrix and blood and parenchyma cells.

The physiologic tissue repair process is traditionally subdivided into 3 phases:
1. Inflammation phase (from 0 to 3 days)
2. Proliferative phase (from 3 to 24 days)
3. Maturation or Epithelialisation phase (from 6-10 days to 12-24 months)

The first phase can be subdivided into the coagulative and inflammatory phases.

The hypertrophic cicatrices and keloids represent an exaggerated response of the biosynthesis of the fibroblasts characterised by abnormal deposits of collagen fibres (collagen hypertrophy) e by a reduced quantity of hyaluronic acid. Hyaluronic acid is a polymer of a saccharide nature that belongs to the chemical family of the glycosaminoglycans (GAGs) made up of repetitive glucuronic and acetylglucosamine acid units. Such macromolecule is abundant in different tissues where it performs specific functions to adjust the remodelling of the cutaneous matrix, to regulate the nutritive exchanges between cells and tissues and to determine cellular migration, proliferation and differentiation.

Attention was given to hyaluronic acid both on an epidermis and dermis level. This macromolecule polysaccharide can be synthesized both by keratinocytes and fibroblasts.

Hyaluronic acid is quickly depolymerised by an enzymatic (hyaluronidase enzyme) and its half-life in the cutis is a few days.

Hyaluronic acid can also be depolymerised by radical. The free radicals, whatever way they are generated by the cutis, can attack the molecule dividing it into smaller sized fragments. As a result of the depolymerisation, whether enzymatic or oxidative, there is a reduction in the dimensions of the molecules and as a direct consequence, a reduction in the capacity of the polya to ligate water. This process lead to a macroscopic drop in the viscoelasticity parameters of the cutis and a reduction of the organisation of the extra cellular matrix characterized by a loss in quality of the collagen fibre that tends to aggregate in increasing dimensioned structures with less elasticity.

Hyaluronic acid is able to prevent the formation of fibrosclerotic tissue and modulates the biosynthesis of collagen on the part of the fibroblasts.

On the basis of an exaggerated response in the formation of hypertrophic cicatrices and/or keloids a chronic inflammation condition is revealed.

Document FR 2 853 539 A discloses a cosmetic composition comprising at least one rhamnose compound, a fucose compound and an excipient.

Document WO 2005/117913 A discloses a pharmaceutical composition to be applied as a topical preparation for treating several diseases which comprises ozonized oils with MSM and dimethylsulfoxide (DMSO) for enhancing penetration of the active principles and permitting the treating of internal and external diseases.

Document WO 02/28400 A discloses a therapeutic formulation for the treatment of osteoarthritis and the maintenance of joint function in animals comprises from 10 to 25% w/v of glucosamine and from 6 to 20% w/v methylsulfonylmethane.

Document EP 0 852 946 A discloses a composition for cosmetic, pharmaceutical or dietetic use and including as the active ingredient, at least one of the substances which include acetylglucosamine and glucuronic acid in combination with the active ingredients which belong to the chemical class of the carboxylic acids, alpha -hydroxy acids, vitamins, amino acids, and bioflavonoids, and formulated with particular synergists, additives, and excipients for external use or for internal use.

Document EP 1 749 532 A discloses a proliferation promoting agent for epidermis keratinized cell comprising N-acetyl-D-glucosamine and a regeneration promoting agent or protecting agent for epithelium, skin or dermis comprising the proliferation promoting agent.

Document WO 03/045366 A discloses a synthesized composition containing zinc ions, calcium ions, rubidium ions and/or potassium ions in a pharmaceutically acceptable carrier, which, when applied to an open wound, effectively modulates the activity of at least MMP-2 and/or MMP-9 in the wound.

PETERSZEGI G ET AL: "Protection by L-fucose and fucose-rich polysaccharides against ROS-produced cell death in presence of ascorbate.", BIOMEDICINE AND PHARMACOTHERAPY, vol. 57, no. 3-4, May 2003 (2003-05), pages 130-133, XP002517047, discloses that fucose and FROP-3 stimulate fibroblast proliferation, protect fibroblast against ROS-produced cell death and have an accelerating effect on would healing.

ISNARD N ET AL: "Studies on corneal wound healing: Effect of fucose on iodine vapor-burnt rabbit corneas" OPHTHALMOLOGICA 200511 CH, vol. 219, no. 6, November 2005 (2005-11), pages 324-333, XP002517048, discloses that fucose decreases the activity of MMP-9 in corneal tissue and can be used for the local treatment of corneal wounds.

### Objective and Summary of the Invention.

The present invention is defined in the appended claims. This invention was conceived starting from the considerations above and its objective is to provide a pharmaceutical or cosmetic composition that has the possibility of intervening directly and simultaneously against the physiology of cicatrisation, by formulating the formation of soluble collagen, blocking cutaneous hypertrophy, facilitating biosynthesis of hyaluronic acid and blocking the inflammatory process.

More precisely, this invention proposes a composition formulated for a combined use of fucose with dimethyl sulfone used for the indications above described in combination with acetylglucosamine.

Compositions are described, which are not covered by the present invention, comprising only one or two of the active ingredients fucose, dimethyl sulfone and acetylglucosamine.

In fact, fucose (and/or FROPs, i.e. Fucose Rich Polysacchairdes) may be used on its own to prevent the formation of hypertrophic scars and/or keloids and to contrast the dimensions when already formed.

Fucose (and/or the FROPs) can be used in a single combination with acetylglucosamine.

Fucose (and/or the FROPs) can be used in a single combination with Dimethyl sulfone.

According to the present invention, dimethyl sulfone and fucose are used in combination with acetylglucosamine.

The composition of this invention may be applied on the skin, whether intact or injured, and used for internal purposes in the pharmaceutical form of tablets, capsules, drops, intramuscular, intravenous, intra-joints, intracutaneous injections etc.

### Detailed Description of the invention

It was surprisingly found that fucose (and/or FROPs) used both individually or in combination with both dimethyl sulfone and acetylglucosamine can exert remarkable activity in the modulating the cicatrisation process. Among other things the combination in the use of fucose (and/or FROPs), of dimethyl sulfone and acetylglucosamine can simultaneously act as a stimulator of the biosynthesis of hyaluronic acid and is able to inhibit both oxidative and enzymatic depolarization.

Fucose (and/or FROPs) specifically stimulates the production of glycosaminoglycans (including hyaluronic acid) and in addition heparin sulphate which is able to inhibit the hyaluronidase enzyme (the enzyme appointed for the depolymerisation of hyaluronic acid), helps in the remodelling of the extra cellular matrix, and stimulates the production of tropocollagen.

Dimethyl sulfone acting as a strong anti-inflammatory, inhibits the allergic response caused by xenobiotics, is able to protect both the keratinocytes and fibroblasts from the detrimental action carried out by ultraviolet radiation, and inhibits the action of cytokines, among which interleukin 1-alpha.

Dimethyl sulfone and fucose (and/or FROPs) carry out a marked antioxidant and anti-free radical activity.

Acetylglucosamine also carries out a marked anti-inflammatory and anti-free radical activity and furthermore, forming one of the two molecules of some glycosaminoglycans (among which hyaluronic acid) it behaves as a stimulator of the biosynthesis of the polysaccharides acting as a biochemical precursor of the same biosynthesis. Acetylglucosamine is also able to act as an efficient antioxidant and to inhibit the depolymerisation of hyaluronic acid mediated by free radicals.

In this invention fucose (and/or FROPs) may be used both in the levorotatory and dextrorotatory forms, and in racemica mixtures, both in the cis and trans forms. Furthermore fucose (and/or FROPs) and Dimethyl sulfone and acetylglucosamine, differently combined between them, can be used in different ponderal ratios.

Fucose (and/or FROPs) may be used individually in a percentage in weight that goes from 0.01% to 90%. Preferably, depending on the form of administration and the pharmaceutical and/or cosmetic form or in a general sense the chemical-physical form used, fucose can be used in a percentage in weight of between 0.2% and 30%. Better still fucose (and/or FROPs) may be used between 0.5% and 10% in weight.

Fucose (and/or FROPs) may be used in a percentage in weight from 0.01% al 90% when associated with dimethyl sulfone with the latter in a percentage in weight from 0.1% to 90%. Preferably depending on the form of administration and the pharmaceutical and/or cosmetic form or in a general sense the chemical-physical form used fucose (and/or FROPs) will be used in a percentage in weight from 0.2% to 30% when dimethyl sulfone is used in a percentage in weight from 0.5% to 50%.

Even more precisely fucose (and/or FROPs) will be used in a percentage in weight from 0.5% to 10% when dimethyl sulfone is used in a percentage in weight from 1% to 20%.

Fucose (and/or FROPs) may be used with a percentage in weight that goes from 0.01% to 90% when associated with acetylglucosamine with the latter in a percentage in weight from 0.01% to 90%. Preferably, depending on the form of administration and the pharmaceutical and/or cosmetic form or in a general sense the chemical-physical form used fucose (and/or FROPs) is used in a percentage in weight from 0.2% to 30% when acetylglucosamine is in a percentage in weight from 0.5% to 30%.

More precisely still fucose (and/or FROPs) will be used in a percentage in weight from 0.5% to 10% when acetylglucosamine is in a percentage in weight from 2% to 10%.

Dimethyl sulfone may be used in a percentage in weight from 0.1% to 90% when associated with acetylglucosamine with the latter in a percentage in weight from 0.01 to 90%. Preferably depending on the form of administration and the pharmaceutical and/or cosmetic form used dimethyl sulfone will be used in a percentage in weight from 0.5% to 50% when acetylglucosamine is in a percentage in weight from 0.5% to 30%.

Still more precisely dimethyl sulfone will be used in a percentage in weight from 1% to 20% when acetylglucosamine is in a percentage in weight from 2% to 10%.

Fucose (and/or FROPs) may be used in a percentage in weight from 0.01% to 90% when associated with dimethyl sulfone with the latter in a percentage in weight from 0.1% to 90% and acetylglucosamine in a percentage in weight from 0.01% to 90%.

Preferably, depending on the form of administration and the pharmaceutical and/or cosmetic form used fucose (and/or FROPs) will be used in a percentage in weight from 0.2% to 30% when dimethyl sulfone is in a percentage in weight from 0.5% to 50% and acetylglucosamine is in a percentage in weight from 0.5% al 30%.

Still more precisely fucose (and/or FROPs) will be used in a percentage in weight from 0.5% al 10% when dimethyl sulfone is in a percentage in weight from 1% to 20% and acetylglucosamine is in a percentage in weight from 2% to 10%.

### EXAMPLES OF FORMULATIONS

### Preparation 1 - reference example

Phial to be injected via intramuscular, intracuttaneous and/or subcutaneous.

| N° | Description | w% |
|---|---|---|
| 01 | Fucose | 0.005 |
| 03 | Dimethyl sulfone | 0.100 |
| 04 | Bidistilled water apirogenic a.r. ml | 100 |

Method of preparation: dissolve the dimethyl sulfone by shaking in water at a temperature of 35°C; when the solution is limpid add the fucose, continue shaking maintaining the temperature constant. Sterilize when solution is limpid.

### Preparation 2

Phial to be injected via intramuscular, intracuttaneous and/or subcutaneously.

| N° | Description | W% |
|---|---|---|
| 01 | Fucose | 0.005 |
| 03 | Dimethyl sulfone | 0.100 |
| 04 | Acetylglucosamine | 0.005 |
| 04 | Bidistilled water apirogenic a.r. ml | 100 |

Method of preparation: dissolve the dimethyl sulfone by shaking in water at a temperature of 35°C; when the solution is limpid add the fucose, continue shaking and add the acetylglucosamine keeping the temperature constant. Sterilize when solution is limpid.

### Preparation 3 - reference example

Phial to be injected via intramuscular, intracuttaneous and/or subcutaneously.

| N° | Description | w% |
|---|---|---|
| 01 | Fucose | 0.005 |
| 02 | Acetylglucosamine | 0.050 |
| 03 | Bidistilled water apirogenic a.r. ml | 100 |

Method of preparation: dissolve the acetylglucosamine by shaking in water at a temperature of 35°C; when the solution is limpid add the fucose, continue shaking maintaining the temperature constant. Sterilize when solution is limpid.

### Preparation 4

### Monophasic Solution

| N° | Description | w% |
|---|---|---|
| 01 | Acetylglucosamine | 1,00 |
| 02 | Fucose | 5,00 |
| 03 | Dimethyl sulfone | 10.00 |
| 04 | Propylene glycol | 5,00 |
| 05 | Glycerol | 2,00 |
| 06 | Dimethyl isosorbide | 10.00 |
| 07 | Preservatives | a.r. |
| 08 | Deionised water a.r. | 100 |

Method of preparation: dissolve the dimethyl sulfone by shaking in water at a temperature of 35°C; when the solution is limpid add acetylglucosamine and fucose; continue shaking maintaining the temperature constant. When solution is limpid mix in the propylene glycol and glycerol, continue to shake.

### Preparation 5 - reference example

### Monophasic solution

| N° | Description | w% |
|---|---|---|
| 01 | Acetylglucosamine | 15,00 |
| 02 | Fucose | 5,00 |
| 03 | Propylene glycol | 5,00 |
| 04 | Glycerol | 2,00 |
| 05 | Dimethyl isosorbide | 10.00 |
| 06 | Preservatives | a.r. |
| 07 | Deionised water a.r. | 100 |

Method of preparation: dissolve the acetylglucosamine by shaking in water at a temperature of 35°C; when solution is limpid add the fucose, continue to shake maintaining the temperature constant.

When solution is limpid mix in the propylene glycol and the glycerol, continue to shake.

### Preparation 6 - reference example

### Monophasic solution

| N° | Description | W% |
|---|---|---|
| 01 | Acetylglucosamine | 15,00 |
| 02 | Dimethyl sulfone | 5,00 |
| 03 | Propylene glycol | 5,00 |
| 04 | Glycerol | 2,00 |
| 05 | Dimethyl isosorbide | 10.00 |
| 06 | Preservatives | a.r. |
| 07 | Deionised water a.r. | 100 |

Method of preparation: dissolve the dimethyl sulfone by shaking in water at a temperature of 35°C when solution is limpid dissolve the acetylglucosamine; continue to shake maintaining the temperature constant.

When solution is limpid mix in the propylene glycol and the glycerol, continue to shake.

### Preparation 7

### Monophasic Gel

| N° | Description | w% |
|---|---|---|
| 01 | Acetylglucosamine | 5,00 |
| 02 | Fucose | 1,00 |
| 03 | Dimethyl sulfone | 3,00 |
| 04 | Dimethyl isosorbide | 20.00 |
| 05 | Preservatives | a.r. |
| 06 | Xanthine gum | 2,00 |
| 07 | Propylene glycol | 3,00 |
| 08 | Ethylenediaminetraacetic acid disodium salt | 0.07 |
| 09 | Deionised water a.r. | 100 |

Method of preparation: dissolve the dimethyl sulfone by shaking in water at a temperature of 35°C; when the solution is limpid dissolve the acetylglucosamine and the fucose, continue to shake and keep the temperature constant. When the solution is limpid mix in the propylene glycol and the glycerol, continue to shake.

Mix in the dimetil isosorbide to the previous solution; when solution is limpid disperse the Xanthine gum. Leave it to shake until a medium viscosity limpid gel is formed.

### Preparation 8 - reference example

### Monophasic Gel

| N° | Description | w% |
|---|---|---|
| 01 | Acetylglucosamine | 5,00 |
| 02 | Dimethyl sulfone | 3,00 |
| 03 | Dimethyl isosorbide | 20.00 |
| 04 | Preservatives | a.r. |
| 05 | Xanthine gum | 2,00 |
| 06 | Propylene glycol | 3,00 |
| 07 | Ethylenediaminetraacetic acid disodium salt | 0.07 |
| 08 | Deionised water a.r. | 100 |

Method of preparation: dissolve the dimethyl sulfone by shaking in water at a temperature of 35°C; when solution is limpid dissolve the acetylglucosamine, continue to shake and maintain the temperature constant. When solution is limpid mix in the propylene glycol and the glycerol, continue to shake.

Mix in the dimetil isosorbide to the previous solution; when solution is limpid disperse the xanthine gum. Leave it to shake until a medium viscosity limpid gel is formed.

### Preparation 9 - reference example

### Monophasic Gel

| N° | Description | w% |
|---|---|---|
| 01 | Acetylglucosamine | 5,00 |
| 02 | Fucose | 1,00 |
| 03 | Dimethyl isosorbide | 20.00 |
| 04 | Preservatives | a.r. |
| 05 | Xanthine gum | 2,00 |
| 06 | Propylene glycol | 3,00 |
| 07 | Ethylenediaminetraacetic acid disodium salt | 0.07 |
| 08 | Deionised water a.r. | 100 |

Method of preparation: dissolve the acetylglucosamine by shaking in water at a temperature of 35°C; when the solution is limpid dissolve the fucose, continue to shake and keep the temperature constant. When the solution is limpid add the propylene glycol and the glycerol, continue to shake.

Mix in the dimetil isosorbide to the previous solution; when solution is limpid disperse the xanthine gum. Leave it to shake until a medium viscosity limpid gel is formed.

### Preparation 10 - reference example

### Monophasic Gel

| N° | Description | w% |
|---|---|---|
| 01 | Dimethyl sulfone | 5,00 |
| 02 | Fucose | 1,00 |
| 03 | Dimethyl isosorbide | 20.00 |
| 04 | Preservatives | a.r. |
| 05 | Xanthine gum | 2,00 |
| 06 | Propylene glycol | 3,00 |
| 07 | Ethylenediaminetraacetic acid disodium salt | 0.07 |
| 08 | Deionised water a.r. | 100 |

Method of preparation: dissolve the dimethyl sulfone by shaking in water at a temperature of 35°C, when solution is limpid dissolve the fucose, always shaking and maintain the temperature constant. When solution is limpid mix in the propylene glycol and the glycerol, always by shaking.

Mix in the dimetil isosorbide to the previous solution; when solution is limpid disperse the xanthine gum. Leave it to shake until a medium viscosity limpid gel is formed.

### Preparation 11 - reference example

### Oil in water emulsion

| N° | Description | w% |
|---|---|---|
| 01 | Polyoxyethylene-2-Stearyl Ether | 3,00 |
| 02 | Polyoxyethylene-21-Stearyl Ether | 2,00 |
| 03 | Octanoic/Decanoic acid triglyceride | 8,00 |
| 04 | 2-Hydroxyethyl Palmitate | 3,00 |
| 05 | Fucose | 2,00 |
| 06 | Deionised water | 10.00 |
| 07 | Glycerol vegetal | 3,00 |
| 08 | Propylene glycol | 2,00 |
| 09 | Preservatives | a.r. |
| 10 | Xanthine gum | 0.50 |
| 11 | Ethylenediaminetraacetic acid disodium salt | 0.10 |
| 12 | Deionised water a.r. | 100 |

Method of preparation: put the polyoxyethylene-2-Stearyl Ether, Polyoxyethylene-21-Stearyl Ether, Octanoic/Decanoic acid triglyceride, 2-Hydroxyethyl Palmitate in a container and shake, heating the content to +80°C. Dissolve the fucose in water, shake and heat to 35°C; Weigh the water in a separate container and, by shaking dissolve the ethylenediaminetetraacetic disodium salt acid in it. When the solution is limpid, shake to mix the glycerol vegetal, propylene glycol and the preservatives.

Dispel the xanthine gum the previous limpid solution.

Allow to shake until a homogenous dispersion has been reached, at a temperature of +80°C.

Using a turbine mix the phases, and bring temperature to +80°C. Cool to +40°C and add, by shaking and using a turbine the phase containing fucose. Continue the cooling operations of the emulsion until the temperature reaches +25°C.

### Preparation 12 - reference example

### Oil in water emulsion

| N° | Description | w% |
|---|---|---|
| 01 | Polyoxyethylene-2-Stearyl Ether | 3,00 |
| 02 | Polyoxyethylene-21-Stearyl Ether | 2,00 |
| 03 | Octanoic/Decanoic acid triglyceride | 8,00 |
| 04 | 2-Hydroxyethyl Palmitate | 3,00 |
| 05 | Dimethyl sulfone | 5,00 |
| 06 | Fucose | 0.50 |
| 07 | Deionised water | 10.00 |
| 08 | Glycerol vegetal | 3,00 |
| 09 | Propylene glycol | 2,00 |
| 10 | Preservatives | a.r. |
| 11 | Xanthine gum | 0.50 |
| 12 | Ethylenediaminetraacetic acid disodium salt | 0.10 |
| 13 | Deionised water a.r. | 100 |

Method of preparation: put the Polyoxyethylene-2-Stearyl Ether, Polyoxyethylene-21-Stearyl Ether, Octanoic/Decanoic acid triglyceride, 2-Hydroxyethyl Palmitate in a container and shake, heating the content to +80°C. Dissolve the dimethyl sulfone in water, shake and heat to 35°C; when the solution is limpid dissolve fucose, always by shaking and maintaining the temperature constant. Weigh the water in a separate container and, by shaking dissolve the ethylenediaminetetraacetic disodium salt acid in it. When the solution is limpid, shake to mix the glycerol vegetal, propylene glycol and the preservatives.

Dispel the xanthine gum the previous limpid solution.

Allow to shake until a homogenous dispersion has been reached, at a temperature of +80°C.

Using a turbine mix the phases, bring temperature to +80°C. Cool to +40°C and add, by shaking and using a turbine the phase containing fucose and dimethyl sulfone. Continue the cooling operations of the emulsion until the temperature reaches +25°C.

### Preparation 13 - reference example

### Oil in water emulsion

| N° | Description | w% |
|---|---|---|
| 01 | Polyoxyethylene-2-Stearyl Ether | 3,00 |
| 02 | Polyoxyethylene-21-Stearyl Ether | 2,00 |
| 03 | Octanoic/Decanoic acid triglyceride | 8,00 |
| 04 | 2-Hydroxyethyl Palmitate | 3,00 |
| 05 | Fucose | 1,50 |
| 06 | Acetylglucosamine | 5,00 |
| 07 | Deionised water | 10.00 |
| 08 | Glycerol vegetal | 3,00 |
| 09 | Propylene glycol | 2,00 |
| 10 | Preservatives | a.r. |
| 11 | Xanthine gum | 0.50 |
| 12 | Ethylenediaminetraacetic acid disodium salt | 0.10 |
| 13 | Deionised water a.r. | 100 |

Method of preparation: put the Polyoxyethylene-2-Stearyl Ether, Polyoxyethylene-21-Stearyl Ether, Octanoic/Decanoic acid triglyceride, 2-Hydroxyethyl Palmitate in a container and shake, heating the content to +80°C. Dissolve the fucose in water, shake and heat to 35°C; when the solution is limpid dissolve the acetylglucosamine, always by shaking and maintaining the temperature constant. Weigh the water in another recipient and, by shaking, dissolve the ethylenediaminetetraacetic disodium salt acid in it. When the solution is limpid, shake to mix the glycerol vegetal, propylene glycol and the preservatives.

Dispel the xanthine gum the previous limpid solution.

Allow to shake until a homogenous dispersion has been reached, at a temperature of +80°C.

Using a turbine mix the phases, bring temperature to +80°C. Cool to +40°C and add, by shaking and using a turbine the phase containing fucose and Acetyl glucosamine. Continue the cooling operations of the emulsion until the temperature reaches +25°C.

### Preparation 14

### Oil in water emulsion

| N° | Description | w% |
|---|---|---|
| 01 | Polyoxyethylene-2-Stearyl Ether | 3,00 |
| 02 | Polyoxyethylene-21-Stearyl Ether | 2,00 |
| 03 | Octanoic/Decanoic acid triglyceride | 8,00 |
| 04 | 2-Hydroxyethyl Palmitate | 3,00 |
| 05 | Fucose | 0.35 |
| 06 | Dimetil sulfone | 3,00 |
| 07 | Acetyl glucosamine | 3,00 |
| 08 | Deionised water | 10.00 |
| 09 | Glycerol vegetal | 3,00 |
| 10 | Propylene glycol | 2,00 |
| 11 | Preservatives | a.r. |
| 12 | Xanthine gum | 0.50 |
| 13 | Ethylenediaminetraacetic acid disodium salt | 0.10 |
| 14 | Deionised water a.r. | 100 |

Method of preparation: put the Polyoxyethylene-2-Stearyl Ether, Polyoxyethylene-21-Stearyl Ether, Octanoic/Decanoic acid triglyceride, 2-Hydroxyethyl Palmitate in a container and shake, heating the content to +80°C. Dissolve the dimethyl sulfone in water, shake and heat to 35°C; when the solution is limpid dissolve fucose, always by shaking and maintaining the temperature constant, when the solution is limpid dissolve the Acetylglucosamine, always by shaking and maintaining the temperature constant. Weigh the water in another container and by shaking, dissolve the Ethylenediaminetraacetic acid disodium salt. When the solution is limpid, shake to mix the glycerol vegetal, propylene glycol and the preservatives.

Dispel the xanthine gum the previous limpid solution.

Allow to shake until a homogenous dispersion has been reached, at a temperature of +80°C.

Using a turbine mix the phases, bring temperature to +80°C. Cool to +40°C and add, by shaking and using a turbine the phase containing fucose and dimethyl sulfone and acetylglucosamine. Continue the cooling operations of the emulsion until the temperature reaches +25°C.

### Preparation 15 - reference example

**Tablets**

| N° | Description | w% |
|---|---|---|
| 01 | Microcrystalline cellulose | 0.50 |
| 02 | Super ventilated talc | 0.02 |
| 03 | Magnesium stearate | 0.01 |
| 04 | Silicon dioxide | 0.014 |
| 05 | Calcium phosphate | 0.30 |
| 06 | Fucose | 60.00 |
| 07 | Hydroxypropylmethylcellulose | 0.01 |
| 08 | Lac | 0.005 |

Method of preparation: the ingredients are loaded into the mixer in a specific order and for a set period. After mixing, the composition is pre-compressed. The composition pre-compressed in this way is fed into the compressor, from which the shaped tablets exit, ready to be covered using an appropriate machine.

### Preparation 16 - reference example

**Tablets**

| N° | Description | w% |
|---|---|---|
| 01 | Microcrystalline cellulose | 0.50 |
| 02 | Super ventilated talc | 0.02 |
| 03 | Magnesium stearate | 0.01 |
| 04 | Silicon dioxide | 0.014 |
| 05 | Calcium phosphate | 0.30 |
| 06 | Dimethyl sulfone | 40.00 |
| 07 | Fucose | 50.00 |
| 08 | Hydroxypropylmethylcellulose | 0.01 |
| 09 | Lac | 0.005 |

Method of preparation: the ingredients are loaded into the mixer in a specific order and for a set period. After mixing, the composition is pre-compressed. The composition pre-compressed in this way is fed into the compressor, from which the shaped tablets exit, ready to be covered using an appropriate machine.

### Preparation 17 - reference example

**Tablets**

| N° | Description | w% |
|---|---|---|
| 01 | Microcrystalline cellulose | 0.50 |
| 02 | Super ventilated talc | 0.02 |
| 03 | Magnesium stearate | 0.01 |
| 04 | Silicon dioxide | 0.014 |
| 05 | Calcium phosphate | 0.30 |
| 06 | Fucose | 40.00 |
| 07 | Acetylglucosamine | 30.00 |
| 08 | Hydroxypropylmethylcellulose | 0.01 |
| 09 | Lac | 0.005 |

Method of preparation: the ingredients are loaded into the mixer in a specific order and for a set period. After mixing, the composition is pre-compressed. The composition pre-compressed in this way is fed into the compressor, from which the shaped tablets exit, ready to be covered using an appropriate machine.

## Claims

1. A pharmaceutical composition for use in the prevention and the treatment of hypertrophic scars and/or keloids, to inhibit any fibrosclerotic processes, **characterised in that** it contains, as an active ingredient, the active principles fucose, dimethyl sulfone and acetyl glucosamine.

2. The composition for use according to claim 1, which contains fucose in a concentration w% between 0.5% and 10%, acetylglucosamine in a concentration w% between 0.2% and 10%, and dimethyl sulfone in a concentration w% between 0.1% and 20%.

3. The composition for use according to claim 1 or 2, wherein the composition is provided in the form of tablets, capsules, drops, intramuscular, intravenous, intra-joint, intracutaneous injections, emulsions, oil in water emulsions, silicon emulsions, monophasic gels, sub micelles biphasic gels, monophasic solutions, powders.

4. A non-therapeutic cosmetic method for the prevention and the treatment of hypertrophic scars and/or keloids, to inhibit any fibrosclerotic processes, **characterised in that** it comprises the administration of a composition containing, as an active ingredient, the active principles fucose, dimethyl sulfone and acetyl glucosamine.

5. The non-therapeutic cosmetic method according to claim 4, in which the composition contains fucose in a concentration w% between 0.5% and 10%, acetylglucosamine in a concentration w% between 0.2% and 10%, and dimethyl sulfone in a concentration w% between 0.1% and 20%.

6. A non-therapeutic cosmetic method according to claim 4 or 5, for cosmetic use of the composition in the form of tablets, capsules, drops, intramuscular, intravenous, intra-joint, intracutaneous injections, emulsions, oil in water emulsions, silicon emulsions, monophasic gels, sub micelles biphasic gels, monophasic solutions, powders.

## Patentansprüche

1. Pharmazeutische Zusammensetzung zur Verwendung bei der Prävention und Behandlung von hypertrophen Narben und/oder Keloiden, zur Hemmung von fibrosklerotischen Prozessen, **dadurch gekennzeichnet, dass** sie die Wirkstoffe Fucose, Dimethylsulfon und Acetylglucosamin als aktive Ingredienzen enthält.

2. Zusammensetzung zur Verwendung nach Anspruch 1, welche Fucose in einer Konzentration w/w zwischen 0,5% und 10%, Acetylglucosamin in einer Konzentration w/w zwischen 0,2% und 10%, und Dimethylsulfon in einer Konzentration w/w zwischen 0,1% und 20% enthält.

3. Zusammensetzung zur Verwendung nach Anspruch 1 oder 2, wobei die Zusammensetzung in Form von Tabletten, Kapseln, Tropfen, intramuskulären, intravenösen, intraartikulären, intradermalen Injektionen, Emulsionen, Öl in Wasser-Emulsionen, Silikon-Emulsionen, monophasischen Gelen, Submizellen-Zweiphasengelen, einphasigen Lösungen und Pulvern bereitgestellt wird.

4. Nicht-therapeutisches kosmetisches Verfahren zur Prävention und Behandlung von hypertrophen Narben und/oder Keloiden zur Hemmung von fibrosklerotischen Prozessen, **dadurch gekennzeichnet, dass** es die Verabreichung einer Zusammensetzung enthaltend die Wirkstoffe Fucose, Dimethylsulfon und Acetylglucosamin als aktive Ingredienzen umfasst.

5. Nicht-therapeutisches kosmetisches Verfahren nach Anspruch 4, bei dem die Zusammensetzung Fucose in einer Konzentration w/w zwischen 0,5% und 10%, Acetylglucosamin in einer Konzentration w/w zwischen 0,2% und 10%, und Dimethylsulfon in einer Konzentration w/w zwischen 0,1% und 20% enthält.

6. Nicht-therapeutisches kosmetisches Verfahren nach Anspruch 4 oder 5, zur kosmetischen Anwendung der Zusammensetzung in Form von Tabletten, Kapseln, Tropfen, intramuskulären, intravenösen, intraartikulären, intradermalen Injektionen, Emulsionen, Öl in Wasser-Emulsionen, Silikon-Emulsionen, monophasischen Gelen, Submizellen-Zweiphasengelen, einphasigen Lösungen und Pulvern.

## Revendications

1. Une composition pharmaceutique destinée à être utilisée dans la prévention et le traitement des cicatrices hypertrophiques et/ou chéloïdes, pour inhiber tout processus fibrosclérotiques, **caractérisée en ce qu'**elle contient, comme ingrédient actif, les principes actifs fucose, methylsulfonylmethane et acétyl-glucosamine.

2. La composition destinée à être utilisée selon la revendication 1, qui contient fucose en une concentration p/p entre 0,5% et 10%, acétyl-glucosamine en une concentration p/p entre 0,2% et 10%, et methylsulfonylmethane en une concentration p/p entre 0,1% et 20%.

3. La composition destinée à être utilisée selon la revendication 1 ou 2, dans laquelle la composition est fournie sous forme de pastilles, capsules, gouttes, injections intramusculaires, intraveineuses, intra-articulaires, intracutanées, émulsions, émulsions huile dans eau, émulsions siliconiques, gels monophasiques, gels biphasiques submicellaires, solutions monophasiques, poudres.

4. Une procédé cosmétique non thérapeutique pour la prévention et le traitement des cicatrices hypertrophiques et/ou chéloïdes, pour inhiber tout processus fibrosclérotique, **caractérisée en ce qu'**il comprend l'administration d'une composition contenant, comme ingrédient actif, les principes actifs fucose, methylsulfonylmethane et acétyl-glucosamine.

5. La procédé cosmétique non thérapeutique selon la revendication 4, dans lequel la composition contient fucose en une concentration p/p entre 0,5% et 10%, acétyl-glucosamine en une concentration p/p entre 0,2% et 10%, et methylsulfonylmethane en une concentration p/p entre 0,1% et 20%.

6. Une procédé cosmétique non thérapeutique selon la revendication 4 ou 5, pour l'utilisation cosmétique de la composition sous forme de pastilles, capsules, gouttes, injections intramusculaires, intraveineuses, intra-articulaires, intracutanées, émulsions, émulsions huile dans eau, émulsions siliconiques, gels monophasiques, gels biphasiques submicellaires, solutions monophasiques, poudres.
